# EUROPÄISCHE PATENTSCHRIFT

(11) **EP 2 291 144 B1**
(45) Veröffentlichungstag und Bekanntmachung des Hinweises auf die Patenterteilung: **09.07.2014**
(21) Anmeldenummer: 09745800.4
(22) Anmeldetag: 14.05.2009
(51) Int. Cl.: A61F 2/16

(54) **IMPLANTIERBARES SYSTEM ZUR HERSTELLUNG DER AKKOMMODATIONSFÄHIGKEIT UNTER NUTZUNG INTERNER ENERGIE**
IMPLANTABLE SYSTEM FOR RESTORING ACCOMMODATION CAPACITY USING INTERNAL ENERGY
SYSTÈME IMPLANTABLE POUR RESTAURER LA CAPACITÉ D'ACCOMMODATION PAR UTILISATION D'ÉNERGIE INTERNE

(30) Priorität: 15.05.2008 DE 102008023726
(43) Veröffentlichungstag der Anmeldung: 09.03.2011
(73) Patentinhaber: Karlsruher Institut für Technologie, 76131 Karlsruhe (DE)
(72) Erfinder: KLINK, Simon, 71063 FINDELFINGEN (DE); BRETTHAUER, Georg, 76139 Karlsruhe (DE); GUTHOFF, Rudolf, 18119 Rostock (DE); BERGEMANN, Mark, 73033 Göppingen (DE)
(74) Vertreter: Fitzner, Uwe
(86) Internationale Anmeldenummer: PCT/EP2009/055854
(87) Internationale Veröffentlichungsnummer: WO 2009/138468

(56) Entgegenhaltungen:
- EP-A1- 1 726 272
- WO-A2-2006/050171
- US-A1- 2005 256 571
- US-A1- 2007 260 307

## Beschreibung

Die vorliegende Erfindung betrifft ein Akkommodationssystem, welches zwecks Wiederherstellung der Akkommodationsfähigkeit unter Nutzung interner Energie in oder am Auge implantierbar ist.

Das menschliche Auge ist ein optisches System, das mit Hilfe mehrerer üchtbrechender Grenzflächen Objekte scharf auf der Netzhaut (retina) abbildet. Hierbei passieren die Lichtwellen die Hornhaut (cornea), das Kammerwasser in der Vorderkammer (camera anterior bulbi), die Linse (lens crystallina) und den Glaskörper in der Hinterkammer (camera vitrea bulbi), die alle unterschiedliche Brechungsindizes aufweisen. Ändert sich die Gegenstandsweite des betrachteten Objektes, ist es für eine Abbildung mit gleich bleibender Schärfe auf der Netzhaut notwendig, das sich das Abbildungsverhalten des optischen Systems ändert. Beim menschlichen Auge wird dies durch eine Verformung der Linse mit Hilfe des Ziliarmuskels (musculus ciliaris) realisiert, wodurch sich im Wesentlichen die Form und die Lage der Linsenvorder- und -rückseite ändern (Akkommodation). Bei einem intakten Akkommodationssystem eines jugendlichen Menschen kann so die Scheitelbrechkraft des Systems zwischen Ferneinstellung (desakkommodierter Zustand) und Naheinstellung (akkommodierter Zustand) um 14 dpt (Akkommodationsbreite) verändert werden. Dadurch können bei einem normalsichtigen (emmetropen) jugendlichen Menschen Objekte, die sich zwischen dem im Unendlichen liegenden Fernpunkt und dem sich in etwa 7 cm vor der Hornhaut liegenden Nahpunkt befinden, scharf auf der Netzhaut abgebildet werden.

Da die Fähigkeit des menschlichen Auges zur Akkommodation mit zunehmendem Alter abnimmt, sind eine Anzahl von künstlich implantierbaren Linsensystemen mit variabler Brennweite entwickelt worden.

Bei potentiell akkommodierenden Intraokularlinsen handelt es sich um Linsen oder Linsensysteme, die nach operativer Entfernung der natürlichen Linse anstelle dieser eingesetzt und vorwiegend im Kapselsack befestigt werden. Durch eine noch vorhandene, jedoch geringe Restkontraktion des Ziliarmuskels, soll über eine Haptik eine axiale Verschiebung der Linse erreicht werden.

Vorrichtungen zur Wiederherstellung der Akkommodationsfähigkeit sind beispielsweise aus der DE 101 55 345 C2, US 66 38 304 B2, WO 03/017873 A1 und US 4373218, DE 94 22 429 U1, DE 201 11 320 U1, DE 100 62 218 A1, DE 10139027, WO 02/083033, DE 10125829 A1, US 2004/0181279A1, US2002/0149743 und US6096078, bekannt.

Ferner gibt es zahlreiche wissenschaftliche Veröffentlichungen zum Thema Akkommodationsfähigkeit von Linsensystemen. Beispielhaft sei auf folgende Veröffentlichungen verwiesen:
Schneider, H.; Stachs, O.; Guthoff, R.: Evidenzbasierte Betrachtungen zu akkommodativen Kunstlinsen 102. Jahrestagung der Deutschen Ophthalmologischen Gesellschaft (Berlin, Germany, September 23rd-26th 2004) (2004)); (Kammann, J.; Dombach, G.: Empirical results regarding accommodative lenses. In: Current Aspects of Human Accommodation. Hrsg.: Guthoff, R.; Ludwig, K. Kaden Verlag Heidelberg (2001) 163-170, Fine, H.; Packer M.; Hoffmann R.: Technology generates IOL with amplitude of accommodation" (Ophtalmology Times Special Report, March 15th 2005) (2005), Lavin, M.: Multifocal intraocular lenses - part 1. Optometry Today 5/2001 (2001) 34-37; Lavin, M.: Multifocal intraocular lenses - part 2. Optometry Today 8/2001 (2001) 43-44. Nishi, O.; Nishi, K.; Mano, C.; Ichihara, M.; Honda, T.: Controlling the capsular shape in lens refilling. Archives of Ophthalmology 115(4) (1997) 507-510; Fine, I.H.: The SmartLens- a fabulous new IOL technology. Eye World 7(10) (2002).

Es wurden bereits Systeme vorgeschlagen, welche die axiale Vorspannung des Kapselsackes nutzen, um unter Ziliarmuskelkontraktion eine Axialbewegung in eine Brechkraftänderung umzusetzen. So wird vorgeschlagen, den Druck des nach hinten ausgelenkten Kapselsackes zu nutzen, um ein Gel teilweise durch eine Lochblende zu drücken und so den Krümmungsradius des auf den anderen Seiten herausquellenden Gels zu beeinflussen. Bei diesem System konnten jedoch bisher die Dauerstabilität eines im Kammerwasser vorhandenen Gels und die ausreichenden optischen Eigenschaften des durch eine Blende gedrückten Gels nicht nachgewiesen werden.

In der DE 199044441 C1 wird vorgeschlagen, sowohl am Kapselsack als auch am Augapfel Magnete anzubringen, um den Kapselsack nach vorne und unter Ziliarmuskelkontraktion noch weiter nach vorne auszulenken. Die im Kapselsack fixierte IOL (Inokularlinse) wird dabei mit bewegt und verursacht dadurch eine Scheitelbrechkraftänderung. Bei diesem System wird der Axialhub durch den Platz im Kammerwasser bis zur Iris begrenzt und reicht nicht aus, um bei einem mit entspanntem Ziliarmuskel emmetropen Auge die notwendige Scheitelbrechkraft zu generieren.

Aus der nicht veröffentlichten DE 102007008 375.2 ist ein aktives mechatronisches System bekannt, welches den Akkommodationsbedarf ermittelt und mit Hilfe einer Elektronik eine Optik variabler Fokuslänge anpasst. Ein solches System mit Messeinheit und Steuerelektronik muss darüber hinaus über ein Energieversorgungssystem verfügen. Die Energieversorgungseinheit muss den laufenden Verbrauch von Messeinrichtung, Steuerelektronik und Optik decken können. Ein für diese Zwecke ausreichendes miniaturisiertes Energieversorgungssystem, welches geeignet ist, den Leistungsbedarf der Elektronik zu decken, ist bisher noch nicht vorhanden.

Die DE 10 2005 038 542 A1 beschreibt eine Vorrichtung zur Wiederherstellung der Akkomodationsfähigkeit umfassend
- wenigstens ein optisches System,
- wenigstens ein Informationserfassungssystem zwecks Erfassung der körpereigenen Steuersignale für Pupillenweite oder Augemotorik oder Akkomodation oder eine Kombination der gesteuerten Steuersignale,
- wenigstens ein Informationsverarbeitungssystem zwecks Erzeugung eines Stellsignals für das optische System aus den erfassten körpereigenen Steuersignalen,
- wenigstens ein Energieversorgungssystem und
- wenigstens ein Befestigungssystem.

Für diese Vorrichtung ist mithin ein Informationserfassungs- und ein Energieversorgungssystem erforderlich.

Die US 6,120,538 offenbart ähnlich wie die DE 10 2005 038 542 A1 ein System mit einer Messdatenverarbeitung (Range-Finder 18 und Controller 16) sowie einer separaten Energiequelle (20). Die US-A-2007/0260307 offenbart ein implantierbares System zur Wiederherstellung der Akkommodationsfähigkeit, bei welchem ein Magnet an einem implantierbaren Ring angeordnet und ein anderer Magnet mit Intraokularlinse verbunden ist.

Nach dem Gesagten ist auf dem Gebiet der Augenheilkunde nach wie vor das Problem zu lösen, dass ab einem Alter von ca. 45 Jahren die Fähigkeit des menschlichen Auges auf einen Leseabstand von ca. 30 cm ausreichend zu akkommodieren (die eigene Linsenbrechkraft anzupassen), abnimmt. Grundsätzlich ist durch die im Rahmen einer Kataraktextraktion implantierte Kunstlinse noch nicht die Möglichkeit geschaffen, auf unterschiedliche Entfernungen zu fokussieren. Bisherige Versuche, intraokulare Strukturen, insbesondere die Ziliarmuskelaktivität im Inneren des Kapselsackes zur mechanischen Brechkraftänderung implantierbarer Systeme zu nutzen, sind aus biologischen Gründen bisher nicht gelungen und mittelfristig ist dies auch nicht zu erwarten.

Es ist daher Aufgabe der vorliegenden Erfindung, ein implantierbares System zur Verfügung zu stellen, welches die Aktivität des Ziliarmuskels umsetzt, aber nicht auf die Verformbarkeit des Kapselsackes angewiesen ist. Außerdem soll ein autarkes System erhalten werden, welches ohne externe Energieversorgung auskommt.

Gelöst wird diese Aufgabe durch ein zur Wiederherstellung der Akkommodationsfähigkeit umfassend wenigstens einen in den Sulcus Ciliaris implantierbaren Ring dadurch gekennzeichnet, dass es wenigstens einen Pattenkondensator, wobei eine Platte am implantierbaren Ring angeordnet ist, und die andere Platte mit dem Kapselsack verbindbar ist, ein in den Kapselsack implantierbares aktiv-optisches Element aufweist, und das aktiv-optische Element und der Kondensator elektrischverbindbar sind.

Vorzugsweise ist die andere Platte mit dem Kapselsack verbunden.

Verwendung findet dieses System demgemäß zur Implantation im oder am Auge zwecks Wiederherstellung der Akkommodationsfähigkeit unter Ausnutzung interner Energie.

Der Ziliarmuskel kontrahiert auch bei presbyopen Personen oder bei jenen, die eine Kataraktoperation hinter sich haben. Dadurch werden die elastisch vorgespannten, radial um den Kapselsack verteilten Zonulafasern entlastet. Unter einer künstlichen Krafteinwirkung auf den Kapselsack in Richtung der optischen Achse ergibt sich unter Ziliarmuskelbewegung eine Verschiebung des Kapselsackes entlang der optischen Achse.

Erfindungsgemäß kann zur Abstützung dieser äußeren Kraft ein in den Sulcus Ciliaris implantierbarer Ring dienen. Der Außendurchmesser des Rings entspricht dem Innendurchmesser des Sulcus Ciliaris und liegt oberhalb von 8 mm. Vorzugsweise liegt der Außendurchmesser des Rings zwischen 8 und 20 mm, besonders bevorzugt zwischen 10 und 18 mm, ganz besonders bevorzugt zwischen 12 und 16 mm, idealerweise bei 14 ± 0,5 mm. Der Innendurchmesser des Rings ist vorzugsweise 0,5 bis 8 mm, weiter bevorzugt zwischen 0,8 und 2,5, weiter bevorzugt zwischen 1,0 und 1,5 mm geringer als der Außendurchmesser.

Infolge der Anordnung des Ringes kann in diesem Bereich vor dem Kapselsack ein Kondensator angebracht werden. Dieser Kondensator ist vorzugsweise ringförmig ausgestaltet. Eine Platte des Kondensators kann am implantierten Ring angeordnet und die andere mechanisch mit dem System im Kapselsack verbindbar bzw. verbunden sein.

Wenn die Kondensatorplatten mit einer definierten Ladung belegt sind, ändert sich mit der Ziliarmuskelbewegung die Position des Kapselsackes entlang der optischen Achse. Dadurch ändert sich auch der Plattenabstand des Kondensators, was eine Spannungsänderung an diesem zur Folge hat.

In den Kapselsack wird ferner ein aktiv-optisches Element implantiert, das elektrisch mit dem Kondensator verbunden wird.Vorzugsweise ist dieses Element ein Electrowetting-Modul. Hierbei erfolgt die Geometrieänderung durch Benetzungswinkelbeeinflussung (Electrowetting): Zwei ineinander nicht mischbare Fluide annähernd gleicher Dichte, die sich in ihren Brechungsindizes unterscheiden, bilden eine sphärisch gekrümmte oder plane Grenzfläche (Meniskus). Wird das eine, elektrisch leitfähige Fluid, in Kontakt mit einer Elektrode gebracht und gegenüber einer zweiten, von den beiden Fluiden durch eine isolierende Schicht (Dielektrikum) getrennte Elektrode eine Potentialdifferenz angelegt, so lässt sich der Benetzungswinkel und somit die Krümmung des Meniskus durch den sog. Elektrowetting-Effekt ändern. Da der Meniskus zwei Medien unterschiedlichen Brechungsindex trennt, wird das optische Abbildungsverhalten verändert. WO99/18456 beschreibt eine axiale Anordnung von leitfähigem Fluid, transparentem Dielektrikum und transparenter Elektrode im Strahlengang und Maßnahmen zur radialen Zentrierung des Tropfens in der optischen Achse. WO03/069380 beschreibt eine Anordnung, bei der die mit einem Dielektrikum beschichtete Elektrode zylindrisch um die optische Achse angeordnet ist. In der optischen Achse befinden sich axial hintereinander angeordnet das elektrisch-leitfähige Fluid und das isolierende Fluid in beliebiger Reihenfolge, sowie der die beiden trennende Meniskus.

Mit dem Electrowetting-Modul kann somit die Krümmung einer sphärischen Grenzfläche zwischen einem leitfähigen und nicht leitfähigen Fluid durch eine Spannungs- oder Ladungsänderung an der vorzugsweise zylinderförmigen Außenfläche beeinflusst werden. Sind die beiden Fluide unterschiedlichen Brechungsindexes, wird dadurch auch die Scheitelbrechkraft des Auges verändert, was der gewünschten Veränderung der Fokuslage gleichkommt. Werden das Electrowetting-Modul und der Kondensator elektrisch verbunden, führt eine Ziliarmuskeibewegung zu einer Veränderung der Fokuslage. Damit kann der ursprüngliche biologische Zusammenhang wiederhergestellt werden. Das erfindungsgemäße System funktioniert dabei, sofern der Kondensator einmal aufgeladen wurde, ohne oder nur mit sehr geringer laufender Energiezufuhr. Der Pattenabstand des Kondensators und dessen Ladung müssen dabei an die Ladungs-/Spannungseigenschaften des Electrowetting-Moduls angepasst werden.

Grundsätzlich eignen sich alle elektrostatischen Aktorkonzepte mit einem geringen Energiezufuhrbedarf, d.h. Konzepte, bei denen vorzugsweise elektrische Ladungen verschoben, aber nicht oder nur in einem sehr geringem Maße verbraucht werden.

An Prinzipien zur Veränderung der Brechkraft stehen zur Verfügung:
1. Elektrowetting
2. Kombinationen aus rein potentialgetriebenen Aktoren ohne dauerhaften Stromfluss, z.B. leitfähiger Polymeraktor, elektrostatischer Polymeraktor, Nanoröhrenraktor DE 10 2004 025 603 A1, Piezokeramikaktor sowie optische Prinzipien, welche durch Verschiebung, Verdrängung oder Verformung usw. basieren, z.B. axialverschiebbare Linse, lateralverschiebbare kubische Linsenpaare, Fluidlinse, elastische Linse sowie alle Systeme, die mit einem oben genannten Aktor betrieben werden können.

Erfindungsgemäß kann eine hohe Ladekapazität beispielsweise durch Einsatz eines Füllmediums mit hoher Dielektrizitätskonstante erreicht werden. Das Füllmedium sollte eine geringe Leitfähigkeit (imaginäre Permittivität), eine hohe reelle Permittivität (=Dielektrizitätskonstante), eine geringe Viskosität und eine hohe Biokompatibilität haben. Beispiele sind Kohlenwasserstoffe wie Dimethylether, Ethanol, Glykol, Propanol ect.. Das Füllmedium ist zur Funktionserfüllung nicht zwingend erforderlich.

Durch die Berührung der Platten kann es zu Kurzschlüssen kommen. Um dies zu vermeiden, können erfindungsgemäß spezielle Vorrichtungen vorgesehen werden. Im einfachsten Falle kann eine Isolation angeordnet sein. Beispielsweise können die Platten mit einer Isolationsschicht versehen sein. Bevorzugt ist z.B. eine Beschichtung aus Keramik oder vorzugsweise Kunststoff. Wesentlich ist auch ein zur Umgebung chemisch inertes Materialverhalten.

Eine andere Variante liegt darin, Distanzstücke einzusetzen, die isolierend, aber auch nicht isolierend sein können.

Durch zusätzliche elastische Elemente z.B. geeignete Federelemente lässt sich die Anziehungskraft zwischen den Kondensatorplatten kompensieren.

### Weitere Federn oder federförmige Elemente dienen der Auslenkung des optischen Systems.

Erfindungsgemäß werden in dem Electrowetting-Modul transparente Materialien ähnlicher Dichte verwendet. Damit sind die optischen Eigenschaften des Systems gesichert. Durch die Wählbarkeit der Kapazität und Ladung des Kondensators kann auch bei kleinem Axialhub stets eine ausreichende Akkommodationsbreite erzielt werden. Durch die Verwendung des Kondensators und die direkte elektrische Verbindung zum Electrowetting-Modul kann dieses System erfindungsgemäß ohne externe Energieversorgung auskommen. Dies bedeutet eine Energieversorgung ausschließlich mit internen Mitteln, wie z.B. mit Solarzellen im Auge oder Schwingelementen mit externer Anregung.

Die Ausführung des künstlichen Akkommodationssystems ohne externe Energieversorgung macht das System in seiner Realisierung einfacher und senkt die Ausfallwahrscheinlichkeit. Es weist dennoch eine sehr gute Optik auf und ist anpassbar an die unterschiedlichen Patienten, so dass eine ausreichende Akkommodationsamplitude gesichert werden kann. Diese Kombination an Vorteilen wird insbesondere durch die Verwendung eines Ringkondensators und die direkte elektrische Verbindung zu einem im Kapselsack positionierten Electrowetting-Modul gewährleistet. Durch den Kraft-Weg-Verlauf der Axialvorspannung des Kapselsackes ist die Umsetzung der Ziliarmuskelkontraktion in einen Akkommodationsbedarf vielfältig vorgebbar.

Im Folgenden wird die Erfindung unter Bezugnahme auf die Figur näher erläutert:

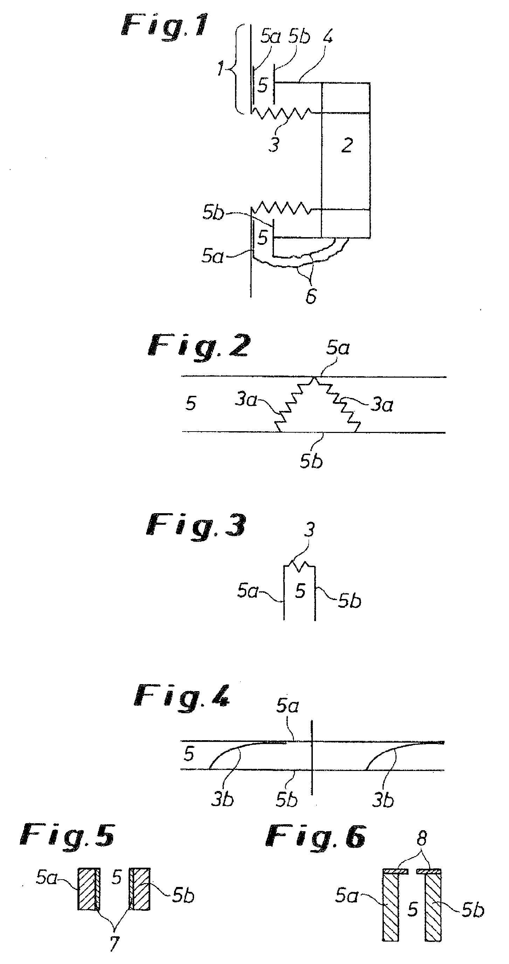

in Figur 1 ist das erfindungsgemäße System schematisch dargestellt. Darin sind der in den Sulcus Ciliaris implantierbare Ring 1 und das Elektrowetting-Modul 2 dargestellt. Alternativ kann es sich hierbei um ein optisches System mit Potenzial getriebenem Aktor handeln.

Das System enthält Federn 3 zur Auslenkung des optischen Systems im Kapselsack nach hinten. Zwischen optischem System und einer Kondensatorplatte 5b des Kondensators 5 besteht eine starre Verbindung 4. Der Kondensator 5 weist einen variablen Plattenabstand auf, wobei die zweite Kondensatorplatte 5a mit dem implantierbaren Ring 1 verbunden ist. Kondensator 5 und optisches System sind über die elektrische Verbindung 6 zusammengeschlossen.

In Figur 2 ist das elastische Element 3a im Detail dargestellt, mit dem sich die Anziehungskraft zwischen den Kondensatorplatten 5a, b des Kondensators 5 kompensieren lässt. Dieses kann als nicht lineare Feder oder in nicht linearer Anordnung zur Vergrößerung des Hubs im Plattenabstand vorliegen.

In Figur 3 ist eine Variante des Federelementes 3a zwischen den Kondensatorplatten 5a, b zur Kompensation der elektrostatischen Anziehung dargestellt.

In Figur 4 sind zirkular verteilte Ringfedern 3b dargestellt.

Zur Vermeidung von Kurzschlüssen sind gemäß Figur 5 zwischen den Kondensatorplatten 5a, b Beschichtungen 7 auf diesen angebracht. Diese Beschichtungen 7 sind elektrisch nicht leitend.

Gemäß Figur 6 ist ein Beispiel für die Anordnung von Distanzstücken 8 gegeben. Diese Distanzstücke 8 können isoliert sein oder selbst aus isolierendem Material bestehen.

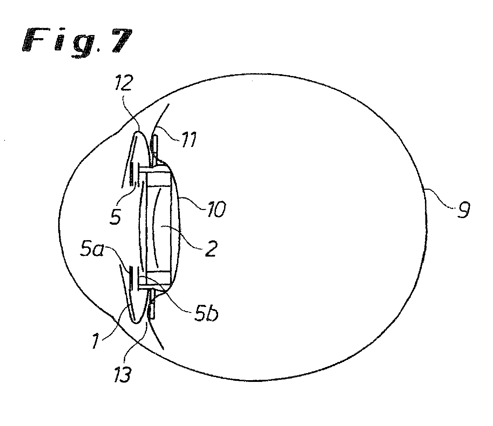

Die Figur 7 stellt den Augapfel 9 mit dem eingesetzten erfindungsgemäßen System dar. Radial um den Kapselsack 10 sind die Zonulafasern 11 verteilt. In den Sulcus Ciliaris 12 ist ein Ring 1 implantiert. An dem Ring 1 ist ein ringförmiger Plattenkondensator 5 angebracht. Eine Platte 5a ist am implantierten Ring 1 und die andere Platte 5b am Kapselsack 10 angeordnet. In den Kapselsack 10 ist ein Electrowetting-Modul 2 implantiert. Werden das Electrowetting-Modul 2 und der Ringkondensator 5 elektrisch verbunden, führt die Bewegung des Ziliarmuskels 13 zu einer Veränderung der Fokuslage.

## Patentansprüche

1. Implantierbares System zur Wiederherstellung der Akkommodationsfähigkeit umfassend wenigstens einen in den Sulcus Ciliaris (12) implantierbaren Ring (1) **dadurch gekennzeichnet,**
**dass** es wenigstens einen Pattenkondensator (5), wobei
- eine Platte (5a) am implantierbaren Ring (1) angeordnet ist, und die andere Platte (5b) mit dem Kapselsack (10) verbindbar ist,
ein in den Kapselsack (10) implantierbares aktiv-optisches Element (2) aufweist, und
- das aktiv-optische Element und der Kondensator (5) elektrischverbindbar sind.

2. Implantierbares System nach Anspruch 1 **dadurch gekennzeichnet, dass** der implantierbare Ring einen Durchmesser von 8 bis 20 mm aufweist.

3. Implantierbares System nach Anspruch 1 **dadurch gekennzeichnet, dass** der Plattenkondensator (5) ringförmig ausgestaltet ist.

4. Implantierbares System nach Anspruch 1 **dadurch gekennzeichnet, dass** es als optisch aktives Element Kombinationen aus rein potentialgetriebenen Aktoren ohne dauerhaften Stromfluss aufweist.

5. Implantierbares System nach Anspruch 1 **dadurch gekennzeichnet, dass** das aktiv-optische Element ein Electrowetting-Modul (2) ist.

6. Implantierbares System nach Anspruch 1 **dadurch gekennzeichnet, dass** es ein Füllmmedium mit hoher Dielektrizitätskonstante aufweist.

7. Implantierbares System nach Anspruch 1 **dadurch gekennzeichnet, dass** es Vorrichtungen (3a,b,7,8) zur Vermeidung von Kurzschlüssen durch Berühren der beiden Kondensatorplatten (5a,b) aufweist.

8. Implantierbares System nach Anspruch 7 **dadurch gekennzeichnet, dass** die Kondensatorplatten (5a,b) mit einer Isolierschicht (7) versehen sind.

9. Implantierbares System nach Anspruch 7 **dadurch gekennzeichnet, dass** Distanzstücke (8) derart angeordnet sind, dass eine Berührung der Kondensatorplatten (5a,b) vermieden wird.

10. Implantierbares System nach der Anspruch 7 **dadurch gekennzeichnet, dass** wenigstens ein Federelement (3a,b), das einer Anziehung der Kondensatorplatten (5a,b) entgegenwirkt, vorgesehen ist.

11. Implantierbares System nach einem der Ansprüche 1 bis 10 **dadurch gekennzeichnet, dass** Federelemente (3) zur Auslenkung des Kapselsacks (10) vorgesehen sind.

## Claims

1. An implantable system for restoring accomodation capacity, comprising at least one ring (1) that can be implanted in the ciliary sulcus (12), caracterized in that
it comprises at least one plate capacitor (5), wherein
- one plate (5a) is arranged on the implantable ring (1) and the other plate (5b) can be connected with the capsular bag (10),
it comprises an active optical element (2) which can be implanted in the capsular bag (10)
- and the active optical element and the capacitor (5) can be electrically connected.

2. The implantable system according to claim 1, caracterized in that the implantable ring has a diameter of 8 to 20 mm.

3. The implantable system according to claim 1, caracterized in that the plate capacitor (5) is ring-shaped.

4. The implantable system according to claim 1, caracterized in that it comprises as active optical elements combinations of strictly potentially powered actuators without permanent current flow.

5. The implantable system according to claim 1 caracterized in that the active optical element is an electrowetting module (2).

6. The implantable system according to claim 1, caracterized in that it has a flling medium with a high dielectric constant.

7. The implantable system according to claim 1, caracterized in that it has devices (3a,b,7,8) for avoiding short-circuits caused by contact between the two capacitor plates (5a,b).

8. The implantable system according to claim 7, caracterized in that the capacitor plates (5a,b) are provided with an insulating layer (7).

9. The implantable system according to claim 7, caracterized in that spacers (8) are arranged in such a way that contact between the capacitor plates (5a,b) is avoided.

10. The implantable system according to claim 7, caracterized in that at least one spring element (3 a,b) is provided to counteract an attraction of the capacitor plates (5 a,b).

11. The implantable system according to one of the claims 1 to 10 , caracterized in that spring elements (3) are provided for deflecting the capsular bag (10).

## Revendications

1. Système implantable pour restaurer la capacité d'accommodation - comprenant au moins un anneau implantable (1) dans le sulcus ciliaire (12) **caractérisé en ce que** le système implantable comprends au moins
un condensateur de plaque (5),
- une plaque (5a) ayant disposée à l'anneau implantable (1) et l'autre plaque (5b) ayant reliable avec le sac capsulaire (10),
un élément optique actif (2) qui est implantable dans le sac capsulaire (10),
- et l'élément optique actif et le condensateur (5) sont reliables électriquement.

2. Système implantable selon la revendication 1, caractérisé en ce l'anneau implantable a un diamètre de 8 à 20 mm.

3. Système implantable selon la revendication 1, **caractérisé en ce que** le condensateur de plaque (5) est fait de manière annulaire.

4. Système implantable selon la revendication 1, **caractérisé en ce qu'**il a comme élément actif optique des combinaisons des acteurs potentiellement actionnées sans conduction de courant permanente.

5. Système implantable selon la revendication 1, **caractérisé en ce que** l'élément optique actif est un module d'électromouillage (2).

6. Système implantable selon la revendication 1, **caractérisé en ce qu'**il présente une medium de remplissage avec une constante diélectrique élevée.

7. Système implantable selon la revendication 1, **caractérisé en ce qu'**il y a des dispositifs (3a,b,7,8) pour éviter des courts-circuits par le contact entre les deux plaques de condensateur (5a,b).

8. Système implantable selon la revendication 7, **caractérisé en ce que** les plaques de condensateur (5a,b) sont fournies avec uns couche isolante (7).

9. Système implantable selon la revendication 7, **caractérisé en ce que** les entretoises (8) sont disposées de telle sorte qu'un contact entre les plaques de condensateur (5a,b) soit evité.

10. Système implantable selon la revendication 7, caractérisé en ce que'au moins un élément de ressort (3 a,b) est prévu que s'oppose à l'attraction des plaques de condensateur (5 a,b).

11. Système implantable selon l'une des revendications 1 à 10, **caractérisé en ce que** les éléments de ressort (3) sont prévus pour le déplacement du sac capsulaire.
